(19) Europäisches Patentamt
European Patent Office
Office européen des brevets

(11) Veröffentlichungsnummer: **0 080 061**
**B1**

# (12) EUROPÄISCHE PATENTSCHRIFT

(45) Veröffentlichungstag der Patentschrift: **06.02.85**

(21) Anmeldenummer: **82109592.4**

(22) Anmeldetag: **18.10.82**

(51) Int. Cl.⁴: **C 07 C 69/76,** C 07 C 65/19, A 61 K 31/235, A 61 K 31/557 // C07F7/18, C07F9/40, C07D317/72, C07C49/743

(54) **Neue cis-Bicyclo(3.3.0)octanderivate, diese enthaltende Arzneimittel und Verfahren zur Herstellung dieser Verbindungen und Arzneimittel.**

(30) Priorität: **21.11.81 DE 3146278**

(43) Veröffentlichungstag der Anmeldung: **01.06.83 Patentblatt 83/22**

(45) Bekanntmachung des Hinweises auf die Patenterteilung: **06.02.85 Patentblatt 85/6**

(84) Benannte Vertragsstaaten: **AT BE CH DE FR GB IT LI LU NL SE**

(56) Entgegenhaltungen:
**EP - A - 0 038 131**
**GB - A - 2 017 699**
**GB - A - 2 070 596**

(73) Patentinhaber: **Grünenthal GmbH, Steinfeldstrasse 2, D-5190 Stolberg (DE)**

(72) Erfinder: **Böhlke, Horst, Dr., Oststrasse 45, D-5190 Stolberg (DE)**
Erfinder: **Loschen, Gerriet, Dr., Erzweg 4, D-5190 Stolberg (DE)**
Erfinder: **Michel, geb. Lambert, Gudrun, Dr., von-Coels-Strasse 98, D-5100 Aachen (DE)**
Erfinder: **Müller, Bernd, Dr., Willemslägerweg 49, D-5106 Roetgen (DE)**

EP 0 080 061 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue cis-Bicyclo-[3.3.0]-octanderivate der Formel I

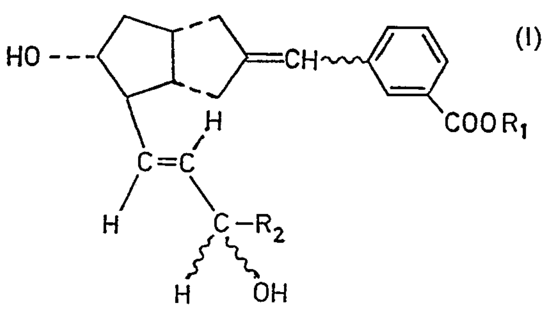

(I)

in denen der Phenylrest in bezug auf die Doppelbindung die EZ- oder E-Konfiguration aufweisen kann und in denen, am u. a. den Rest $R_2$ tragenden Kohlenstoffatom, die RS- oder S-Konfiguration vorliegt.

In der Formel I bedeuten

$R_1$ ein Wasserstoffatom, einen geradkettigen oder verzweigten Alkylrest mit 1 bis 4 Kohlenstoffatomen, oder ein pharmazeutisch verträgliches Kation, und

$R_2$ einen Cyclohexylrest, einen 4-Methylcyclohexylrest oder einen 1-Adamantylrest der Struktur

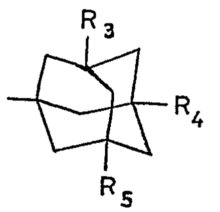

in der $R_3$, $R_4$ und $R_5$ gleich oder verschieden sind und für Wasserstoff oder Methyl stehen.

Vorzugsweise steht $R_1$ für ein Wasserstoffatom, eine Methyl- oder Ethylgruppe oder für ein Natrium- oder Kaliumion. Weitere geeignete Kationen sind insbesondere aus der Prostaglandin- bzw. Prostacyclinchemie bekannt.

Vorzugsweise steht $R_2$ für den Rest

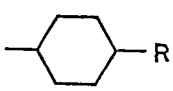

worin R Wasserstoff oder Methyl bedeutet, und eine besonders bevorzugte durch $R_2$ dargestellte Gruppe ist der unsubstituierte Cyclohexylrest.

Die Verbindungen der Formel I haben wertvolle pharmakologische Wirkungen. So wirken sie insbesondere thrombozytenaggregationshemmend, daneben aber auch blutdrucksenkend, und entsprechen damit dem Wirkungstyp z.B. des Prostacyclins, zeichnen sich aber gegenüber dem Prostacyclin z.B. durch eine erheblich gesteigerte Stabilität aus.

Es sind zwar schon chemisch relativ stabile Analoga des Prostacyclins wie z.B. Carbacyclin (das sich wie die Verbindungen der Formel I vom cis-Bicyclo-[3.3.0]-octan ableitet) beschrieben worden, jedoch haben auch diese Verbindungen *in vivo* wie Prostacyclin nur eine kurze Wirkdauer (vgl. z.B. Whittle *et al.*, „Prostaglandins", *19* [1980], S. 605 bis 627, insbesondere S. 623). Im Vergleich zu diesen vorbekannten Substanzen zeigen die Verbindungen der Formel I überraschenderweise nicht nur eine wesentlich länger anhaltende Wirkung, sondern auch einen erheblich grösseren Abstand zwischen den die Aggregationshemmung bzw. die Blutdrucksenkung bewirkenden Dosen. Damit sind die Verbindungen der Formel I sowohl bei Krankheitszuständen verwendbar, bei denen eine Aggregationshemmung ohne begleitende Blutdrucksenkung erwünscht ist (z.B. Hyperaggregabilität bei koronarer Herzkrankheit), als auch in höheren Dosen bei Krankheitszuständen, bei denen eine begleitende gefässerweiternde (blutdrucksenkende) Wirkung neben der Thrombozytenaggregationshemmung zweckmässig ist (z.B. periphere arterielle Verschlusskrankheiten). Die beschriebenen Effekte lassen sich z.B. mit den unten wiedergegebenen experimentell gefundenen Werten belegen.

Aus der belgischen Patentschrift Nr. 887721 (Beispiel 13) ist die E- bzw. die Z-Form des 1,5-Inter-m-phenylen-2,3,4-trinorcarbacyclins, d.h. des 3-(m-Carboxybenzyliden)-6β-(3'S-hydroxy-1'E-octenyl)-7α-hydroxy-cis-bicyclo-[3.3.0]-octans bekannt. Die EZ-Form dieser Verbindung (nachstehend als Substanz A bezeichnet) wurde in einigen der hier beschriebenen Versuche als weitere Vergleichssubstanz herangezogen, wobei sich überraschenderweise eine überlegene Wirkung für die Verbindungen der Formel I, insbesondere derjenigen, in denen $R_2$ einen Cyclohexylrest darstellt, auch gegenüber der Substanz A zeigte.

Das Enzym Adenylatcyclase katalysiert die Bildung von cyclischem 3',5'-Adenosinmonophosphat (c-AMP) aus Adenosintriphosphat (ATP). Wird durch Substanzen wie z.B. Prostacyclin die Adenylatcyclase in Thrombozyten stimuliert, so kommt es zu einer beachtlichen Erhöhung des c-AMP-Spiegels in den Thrombozyten, wodurch eine Aggregation der Thrombozyten unterbunden wird. Die Tabelle 1 gibt das Ausmass der Stimulation der Adenylatcyclase in Pferdethrombozyten durch einige der Verbindungen der Formel I im Vergleich zu dem durch 5,6-Dihydroprostacyclin bzw. Substanz A ausgelösten Effekt an.

*Tabelle 1*

| Substanz | $EC^{*}_{200\%}$ (μMol/l) | relative Wirksamkeit |
|---|---|---|
| 5,6-Dihydroprostacyclin | 15,0 | 1,0 |
| Substanz A | 0,40 | 37,5 |
| Beispiel 1e | 0,15 | 100,0 |
| Beispiel 2 | 0,13 | 115,4 |
| Beispiel 3e | 0,18 | 83,3 |

* Dosis, die die 3fache Stimulation der c-AMP-Bildung aus ATP bewirkt

Die durch Arachidonsäure *in vitro* bewirkte Aggregation von Humanthrombozyten kann z.B. durch Prostacyclin verhindert werden. Die Tabelle 2 nennt den $IC_{50}$-Wert (d.h. die Konzentration, die unter den Versuchsbedingungen eine 50%ige Hemmung der Thrombozytenaggregation bewirkt) und die auf die Wirkung des Prostacyclins bezogene relative Wirksamkeit für einige bekannte Substanzen sowie für die Produkte der Beispiele 1e und 2.

*Tabelle 2*

| Substanz | $IC_{50}$ (µMol/l) | relative Wirksamkeit |
|---|---|---|
| Prostacyclin | 0,0078 | 1,0 |
| 5,6-Dihydroprostacyclin | 0,18 | 0,043 |
| Substanz A | 0,12 | 0,065 |
| Beispiel 1e | 0,08 | 0,098 |
| Beispiel 2 | 0,07 | 0,11 |

In diesem Zusammenhang sei darauf hingewiesen, dass Whittle *et al.* (*loc. cit.*, S. 611) für Carbacyclin fanden, dass dieses als Inhibitor der Thrombozytenaggregation *in vitro* für die durch Arachidonsäure induzierte Aggregation nur das 0,02fache der Prostacyclinwirkung zeigt.

Tabelle 3 gibt die Wirksamkeit einiger Verbindungen gegenüber der ADP-induzierten Thrombozytopenie *in vivo* an der narkotisierten Ratte (Urethannarkose) bei intravenöser Verabreichung der Prüfsubstanzen an.

*Tabelle 3*

| Substanz | relative Wirksamkeit |
|---|---|
| Dihydroprostacyclin | 1,0 |
| Substanz A | 0,1 |
| Beispiel 1e | 1,0 |
| Beispiel 2 | 1,0 |

Aus den Tabellen 1 bis 3 ergibt sich die gute thrombozytenaggregationshemmende Wirkung der Substanzen der Formel I. Wie bereits gesagt, wirken diese aber erst in höherer Dosierung blutdrucksenkend, wie sich aus Tabellen 4a und 4b ergibt (darin ist die $ED_{20}$ diejenige Dosis, die eine Senkung des diastolischen Blutdrucks um 20 mmHg bewirkt).

*Tabelle 4a*

*Blutdrucksenkende Wirkung an wachen, spontan hypertonen Ratten (Messung über Dauerkatheter, intravenöse Applikation der Prüfsubstanzen)*

| Substanz | $ED_{20}$ (mg/kg) | relative Wirksamkeit |
|---|---|---|
| 5,6-Dihydroprostacyclin | 0,005 | 1,0 |
| Substanz A | 1,9 | 0,003 |
| Beispiel 1e | 0,073 | 0,068 |
| Beispiel 2 | 0,094 | 0,053 |
| Beispiel 3e | >1,0 | <0,005 |

*Tabelle 4b*

*Blutdrucksenkende Wirkung an mit Pentobarbital narkotisierten Ratten (intravenöse Applikation der Prüfsubstanzen)*

| Substanz | $ED_{20}$ (µg/kg) | relative Wirksamkeit |
|---|---|---|
| Prostacyclin | 0,16 | 1,0 |
| Beispiel 2 | 5,39 | 0,03 |

Prostacyclin und Carbacyclin werden *in vivo* schnell zu unwirksamen Produkten metabolisiert und zeigen daher nur eine sehr kurze Wirkdauer. Im Vergleich dazu wirken die Verbindungen der Formel I erheblich länger, wie mit folgenden Tabellen gezeigt werden kann.

*Tabelle 5*

Nach intravenöser Applikation einer supramaximalen hypotensiven Dosis (1 mm/kg) an mit Pentobarbital narkotisierten Ratten zeigte sich folgende Abklingcharakteristik der blutdrucksenkenden Wirkung:

| Substanz | Abklingquote des blutdrucksenkenden Effektes (%/min) | Halbierungszeit des blutdrucksenkenden Effektes (min) |
|---|---|---|
| Prostacyclin | 11,0 | 5,89 |
| Beispiel 2 | 2,6 | 25,96 |

Daraus ergibt sich, dass das Produkt des Beispiels 2 etwa 5mal länger wirkt als Prostacyclin. Auch im Hinblick auf die thrombozytenaggregationshemmende Wirkung konnte die wesentlich längere Wirkdauer des Produktes des Beispiels 2 experimentell gezeigt werden. Dazu wurde die Substanz in einer Dosis von 4,64 mg/kg oral an mit Urethan narkotisierte Ratten verabreicht und dann der Zeitverlauf der thrombozytenaggregationshemmenden Wirkung (ADP-Aggregation *in vivo*, Messparameter: ADP-induzierter Abfall der Thrombozytenzahl, gemessen mit Technicon Autocounter) bestimmt. Die folgende Tabelle gibt die prozentuale Aggregationshemmung zu den verschiedenen Zeitpunkten der Messung an:

| | Zeit nach Applikation (min) | | |
|---|---|---|---|
| | 30 | 60 | 150 |
| Aggregationshemmung (%) | 38 | 26 | 22 |

Nach oraler Gabe war also eine wenigstens über 2,5 h anhaltende ausgeprägte thrombozytenaggregationshemmende Wirkung des Produktes aus Beispiel 2 zu beobachten, während z.B. von Prostacyclin bekannt ist, dass es nach oraler Gabe unwirksam ist.

Da die erfindungsgemässen Substanzen der Formel I sich nicht nur durch überraschende wertvolle biologische Eigenschaften, sondern auch durch gute chemische Stabilität auszeichnen, eignen sie sich sowohl zur parenteralen als auch zur oralen Verabreichung zur Hemmung der Thrombozytenaggregation am Menschen, und zwar zur Vorbeugung und Behandlung von Krankheitszuständen, bei denen die Thrombozytenaggregation und/oder eine Hyperaggregabilität pathogenetisch von Bedeutung sind. Solche Krankheitsbilder sind z.B. arterielle Thrombosen bei Endothelläsionen, Atherosklerose, hämostatische arterielle und venöse Thrombosen sowie der Myokardinfarkt. Aufgrund ihrer Wirkung auf den Blutdruck kommen die Verbindungen der Formel I auch zur Behandlung von pulmonalem sowie systemischem Hochdruck in Betracht. Vorteilhaft sind die erfindungsgemässen Substanzen auch zur Verminderung der Aggregabilität bei extrakorporalen Blutkreisläufen (künstliche Niere, Herz-Lungen-Maschine etc.) einsetzbar, wobei man die Substanzen in mikromolaren Konzentrationen dem Patientenblut zusetzt.

Die erfindungsgemässen Substanzen bewirken ferner eine Verminderung der Magensäuresekretion. Sie kommen daher auch zur Behandlung von Erkrankungen mit erhöhter Magensäuresekretion wie z.B. Magen- und Darmulcera, ausserdem aber auch zur Therapie von Ulcuserkrankungen anderer Genese, wie z.B. Antiphlogisticaulcera, in Betracht.

Die Erfindung betrifft dementsprechend auch Arzneimittel, die als Wirkstoff eine oder mehrere der erfindungsgemässen Verbindungen enthalten. Der Wirkstoffgehalt pro Einzeldosis beträgt dabei 0,01 bis 50 mg, und zwar bei Zubereitungsformen für die parenterale Applikation 0,01 bis 10 mg und bei Zubereitungen für die orale Applikation 0,1 bis 50 mg. Arzneimittel zur parenteralen Applikation können sowohl Lösungen als auch Suspensionen oder auch Sprays, z.B. zur intranasalen Anwendung, darstellen, es kommen aber auch leicht rekonstituierbare Trockenzubereitungen in Betracht, wie z.B. in Einzelabpackung lyophilisierte Natriumsalze der Verbindungen der Formel I.

Für die orale Verabreichung kommen Tabletten, Dragees oder Kapseln in Betracht, wobei die Wirkstoffe gegebenenfalls mit üblichen Trägermaterialien, Tablettensprengmitteln, Bindemitteln usw. vermischt bzw. verarbeitet sind. Solche oralen Verabreichungsformen können auch in üblicher Weise so hergestellt werden, dass sie den Wirkstoff verzögert freisetzen, um so über einen längeren Zeitraum eine gleichförmige Versorgung des Patienten mit dem Wirkstoff zu gewährleisten. In diesem Fall ist es möglich, die vorstehend genannte Einzeldosis zu erhöhen, beispielsweise auf 100 oder 250 mg pro Einzelform der Retardzubereitung, vorausgesetzt, dass die Freigaberate so ist, dass die Blutspiegel an Wirkstoffen nicht höher liegen als bei oraler Verabreichung einer nichtretardierten Zubereitungsform.

Die erfindungsgemässe Herstellung dieser Arzneimittel erfolgt in an sich bekannter Weise, wobei selbstverständlich bei der Herstellung der parenteral anzuwendenden Zubereitungen auf Sterilität und – wenn sie in flüssiger Form vorliegen – Isotonie zu achten ist.

Die Erfindung bezieht sich weiterhin auf die Herstellung der Verbindungen der Formel I durch Umsetzung einer Verbindung der allgemeinen Formel II

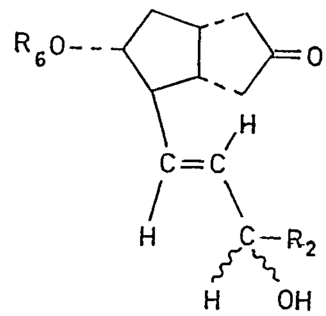

(II)

worin $R_2$ die gleiche Bedeutung wie oben hat und $R_6$ für ein Wasserstoffatom oder eine unter milden Bedingungen abspaltbare Schutzgruppe steht, mit einer Verbindung der Formel III

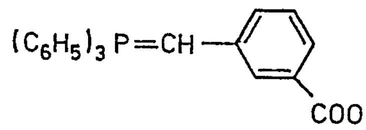

(III)

worin $R_1$ die gleiche Bedeutung wie oben hat, und gegebenenfalls anschliessendes Abspalten der Schutzgruppe, wenn $R_6$ von Wasserstoff verschieden ist. Bevorzugte in der Verbindung der Formel II durch $R_6$ dargestellte Schutzgruppen sind der Tetrahydropyranylrest oder ein Trialkylsilylrest, der in den Alkylresten insgesamt 3 bis 6 Kohlenstoffatome enthält, es kommen aber z.B. auch der Benzoyl- oder der p-Phenylbenzoylrest und weitere, in der Prostaglandinchemie gebräuchliche Schutzgruppen in Betracht.

Die Umsetzung der Verbindung der allgemeinen Formel II mit der Verbindung der Formel III erfolgt unter sorgfältigem Feuchtigkeitsausschluss und unter einer Schutzgasatmosphäre (geeignete Schutzgase sind insbesondere Argon oder Stickstoff) in Gegenwart inerter, aprotischer Lösungsmittel wie z.B. Benzol, Toluol, Dimethylsulfoxid oder Dimethylformamid bei Temperaturen von etwa 0 bis etwa 100°C, vorzugsweise in Benzol, und bei Temperaturen von 20 bis 60°C und zweckmässig unter Zusatz einer geringen Menge einer schwach sauren Verbindung wie z.B. Thiophenol, p-Chlorthiophenol, Thiokresol und ähnlichen Substanzen.

Man erhält so die Verbindung der Formel I in Form des EZ-Isomerengemisches. Falls darin $R_1$ einen Alkylrest darstellt, kann die Gruppe $COOR_1$, gegebenenfalls nach vorheriger Isomerentrennung, zur Carboxylgruppe ($R_1$ = H) verseift und das so erhaltene Produkt dann gewünschtenfalls in ein pharmazeutisch verträgliches Salz übergeführt werden. Man kann aber auch zunächst die Verseifung bzw. Salzbildung durchführen und dann erst die Isomeren trennen. Steht jedoch $R_1$ für Wasserstoff und werden Produkte der Formel I angestrebt, in denen $R_1$ einen wie oben definierten Alkylrest bedeutet, so kann man das zunächst erhaltene Produkt, gegebenenfalls nach vorheriger Isomerentrennung, in üblicher Weise verestern.

Die Trennung der E- und Z-Isomeren erfolgt mit Hilfe der Hochdruckflüssigkeitschromatographie (HPLC) auf übliche Weise unter Verwendung beispielsweise von Methanol/Wasser (80:20) als Laufmittel.

Die Phosphinalkylene der allgemeinen Formel III werden durch Umsetzung eines in an sich bekannter Weise (vgl. z.B. Houben-Weyl, „Methoden der organischen Chemie", Bd. 5/4, Stuttgart 1960, S. 337, oder R.C. Fuson *et al.* „J. Am. Chem. Soc.", *62* [1940], 1180) gewonnenen Phosphoniumsalzes der Formel

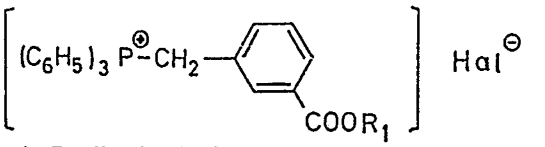

worin $R_1$ die gleiche Bedeutung wie oben hat und Hal z.B. Chlor oder Brom bedeutet,
mit einer starken Base wie Kalium-tert.-butylat, Methansulfinylnatrium, -kalium oder -lithium oder insbesondere Natriumbis(trimethylsilyl)amid hergestellt, wobei man in sorgfältig getrockneten Lösungsmitteln wie z.B. Benzol, Toluol, Dimethylsulfoxid, Hexamethylphosphorsäuretriamid, Tetrahydrofuran oder dergleichen bei Temperaturen von 0 bis etwa 100, vorzugsweise 20 bis 80°C, arbeitet. Dabei ist es nicht erforderlich, die Verbindungen der Formel III vor der Umsetzung mit der Verbindung der Formel II zu isolieren.

Die Herstellung der Ausgangsverbindungen der allgemeinen Formel II, in denen $R_6$ für Wasserstoff steht, erfolgt nach der schematisch in den Formeln II(RS), I(S) und IV bis VIII dargestellten Reaktionsfolge (wobei in den dort gebrauchten Formeln jeweils Z mit Ausnahme von Wasserstoff die gleiche Bedeutung wie $R_6$ und $R_2$ die gleiche Bedeutung wie oben definiert haben).

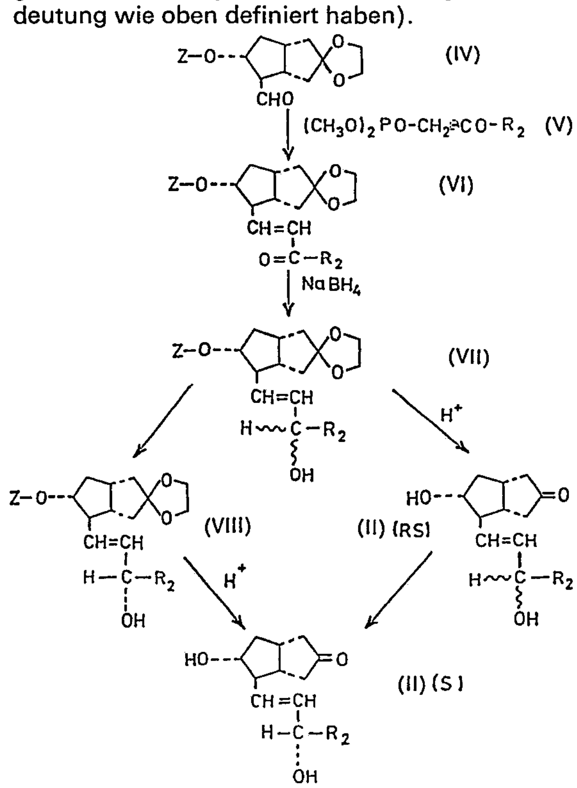


Dazu seien noch folgende Erläuterungen gegeben:

Der Aldehyd IV mit z.B. Z = tert.-Butyldimethylsilyl, also das 3,3-Ethylendioxy-6β-formyl-7α-tert.-butyldimethylsilyloxy-cis-bicyclo-[3.3.0]-octan (Nicolaou *et al.*, „J. Chem. Soc.", Chem. Commun. *1978*, 1067) wird in an sich bekannter Weise mit einem Phosphonat der allgemeinen Formel V zu einem Keton der allgemeinen Formel VI umgesetzt. Die Reaktion erfolgt unter sorgfältigem Ausschluss von Feuchtigkeit und unter Schutzgasatmosphäre wie Argon oder Stickstoff. Als Lösungsmittel dienen z.B. Benzol, Toluol, Dimethoxyethan, Dioxan oder Tetrahydrofuran. Die Reaktion erfolgt bei Temperaturen zwischen etwa −20 und 100, vorzugsweise zwischen 0 und 25°C, im Laufe eines Zeitraumes von etwa 2 bis 24 h.

Die Reduktion der so erhaltenen Ketoverbindungen VI erfolgt durch Umsetzung mit komplexen Borhydriden wie z.B. mit Natriumborhydrid, wobei die Zugabe von Cer-III-salzen vorteilhaft ist. Die Reaktion erfolgt zwischen −40 und 60, vorzugsweise zwischen 0 und 25°C.

Das erhaltene Epimerengemisch VII kann durch Säulenchromatographie an Kieselgel 60 mit einem geeigneten Laufmittelgemisch in die S- bzw. die R-Formen getrennt werden, wobei letztere hier ohne Interesse sind.

Die Abspaltung der Schutzgruppen, d.h. der Ketalgruppe und des für Z bevorzugten tert.-Butyldimethylsilylrestes, erfolgt im essigsauren Medium (Eisessig/Wasser/Tetrahydrofuran 3:1:1) bei etwa 25°C (Corey *et al.*, „J. Amer. Chem. Soc.", *94*, 6190 [1972], Nicolaou *et al.*, „J. Chem. Soc.", Chem. Commun. *1978*, 1067). Man erhält so die S-Formen der Verbindungen der allgemeinen Formel II.

Es ist aber auch möglich, vom Epimerengemisch VII zunächst die Schutzgruppen zu entfernen und dann das Epimerengemisch der allgemeinen Formel II(RS) durch Säulenchromatographie z.B. an Kieselgel 60 zur Gewinnung des Isomeren II(S) zu trennen. Zur Herstellung der 3'-RS-Formen der Verbindungen der Formel I kann man natürlich auch vom Isomerengemisch II(RS), gegebenenfalls nach Einführung einer Schutzgruppe $R_6$, ausgehen und dieses mit der Verbindung der Formel III umsetzen.

Zur Herstellung einer Verbindung der Formel II, in der der Rest $R_6$ eine der definierten Schutzgruppen ist, kann man z.B. in einer Verbindung der Formel VII oder VIII die Ethylendioxygruppe selektiv spalten. Man kann aber auch so vorgehen, dass man in eine Verbindung der Formel II, in der $R_6$ Wasserstoff ist, in üblicher Weise eine Schutzgruppe $R_6$ einführt.

Die folgenden Beispiele dienen zur näheren Erläuterung der Erfindung. Bei ihrer Durchführung wurde auf die Erzielung maximaler Ausbeuten kein Wert gelegt. Alle Temperaturangaben sind unkorrigiert.

Die Reaktionen wurden im Dünnschichtchromatogramm (DC) verfolgt (Fertigplatten, Kieselgel 60, E. Merck).

Für die Säulenchromatographie wurde, soweit

nichts anderes erwähnt, Kieselgel 60, Korngrösse 0,040 bis 0,063 mm (230 bis 400 mesh ASTM) von Macherey-Nagel benutzt.

Die Mischungsverhältnisse der Laufmittel für die Chromatographieverfahren verstehen sich Volumen/Volumen.

Ether (hierunter wird im folgenden stets der Diethylether verstanden) wurde vor der Verwendung in Laufmittelgemischen für die Säulenchromatographie destilliert.

Die $^{1}H$-NMR-Spektren wurden bei 60 MHz und die $^{13}C$-NMR-Spektren bei 15,08 MHz mit dem Gerät WP-60 der Firma Bruker aufgenommen. Die chemische Verschiebung wurde in ppm angegeben.

*Beispiel 1:*

a) *3,3-Ethylendioxy-6β-(3'-oxo-3'-cyclohexyl-1'E-propenyl)-7α-tert.-butyldimethylsilyloxy-cis-bicyclo-[3.3.0]-octan*

Allgemeine Formel VI: $R_2$ = Cyclohexyl; Z = tert.-Butyldimethylsilyl

Zu einer Suspension von 28,3 mg Natriumhydriddispersion (50%ig in Öl) in 15 ml absolutem Dimethoxyethan gibt man unter Rühren und unter Argon als Schutzgas bei Raumtemperatur tropfenweise die Lösung von 139 mg 2-Oxo-2-cyclohexylethanphosphonsäuredimethylester in 2 ml absolutem Dimethoxyethan und rührt 45 min bei Raumtemperatur nach. Dann fügt man tropfenweise die Lösung von 194 mg 3,3-Ethylendioxy-6β-formyl-7α-tert.-butyldimethylsilyloxy-cis-bicyclo-[3.3.0]-octan in 1 ml absolutem Dimethoxyethan hinzu und rührt weiter unter Argonschutz und bei Raumtemperatur. Nach etwa 3 h gibt man die Lösung von 34,4 µl Eisessig in 400 µl Ether zu und engt das Gemisch im Vakuum ein. Das erhaltene, schwach gelbliche Öl nimmt man in 10 ml Ether auf, wäscht die etherische Lösung mit 5 ml gesättigter Natriumhydrogencarbonatlösung sowie mit 5 ml gesättigter Natriumchloridlösung, trocknet über Natriumsulfat und engt die Lösung im Vakuum ein.

Der ölige Rückstand wird durch Säulenchromatographie gereinigt. Mit Ether/Hexan (3:2) erhält man 188 mg (= 73% d. Th.) der Titelverbindung.

$C_{25}H_{42}SiO_4$: 434,702

$^{1}H$-NMR ($CDCl_3$): 6,75 (2d, 1H), 6,15 (d, 1H), 3,93 (s, 4H), 3,89 (s, 1H), 0,95 (s, 9H), 0,8 (s, 6H).

b) *3,3-Ethylendioxy-6β-(3'S-hydroxy-3'-cyclohexyl-1'E-propenyl)-7α-tert.-butyldimethylsilyloxy-cis-bicyclo-[3.3.0]-octan*

Allgemeine Formel VIII: $R_2$ = Cyclohexyl; Z = tert.-Butyldimethylsilyl

Zu einer Lösung von 1,36 g des nach Beispiel 1a erhaltenen Produktes in 18 ml Methanol gibt man 7,8 ml einer 0,4M methanolischen Cer-III-chloridlösung und trägt unter Rühren und Kühlen mit Eis in kleinen Portionen 118,5 mg Natriumborhydrid ein. Nach beendeter Zugabe rührt man noch 5 min unter Eiskühlung und lässt dann bei Raumtemperatur nachrühren. Nach etwa 30 min rührt man 31 ml pH 7 Puffer ein, versetzt mit etwa 80 ml Methylenchlorid sowie 50 ml gesättigter Kaliumnatriumtartratlösung, trennt die organische Phase ab und extrahiert noch 3mal mit jeweils etwa 50 ml Methylenchlorid. Die vereinigten Methylenchloridlösungen wäscht man mit etwa 25 ml gesättigter Natriumchloridlösung, trocknet die organische Phase über Natriumsulfat und engt die Lösung im Vakuum ein.

Der ölige Rückstand wird durch Säulenchromatographie gereinigt. Mit Ether/Petrolether (1:1) erhält man 516,2 mg (= 38% d. Th.) der Titelverbindung, 111,4 mg (= 8% d. Th.) des entsprechenden 3'R-Isomeren sowie 472,3 mg (= 35% d. Th.) des 3'RS-Isomerengemisches, das durch erneute Chromatographie weiter aufgetrennt werden kann.

$C_{25}H_{44}SiO_4$: 436,718

$^{1}H$-NMR ($CDCl_3$): 5,48 (m, 2H), 3,90 (s, 4H), 3,76 (m, 2H), 0,91 (s, 9H), 0,6 (s, 6H).

c) *6β-3'-S-Hydroxy-3'-cyclohexyl-1'E-propenyl)-7α-hydroxy-cis-bicyclo-[3.3.0]-octan-3-on*

Allgemeine Formel II: $R_2$ = Cyclohexyl

516,2 mg der nach Beispiel 1b gewonnenen Substanz gibt man in 5 ml eines Gemisches aus Eisessig/Tetrahydrofuran/Wasser (3:1:1) und rührt etwa 20 h bei Raumtemperatur. (Zur Reaktionskontrolle verwendet man als DC-Laufmittel Ether/Aceton 2:1.) Danach versetzt man vorsichtig mit 20 ml gesättigter Natriumhydrogencarbonatlösung sowie mit festem Natriumhydrogencarbonat bis die Essigsäure neutralisiert ist, extrahiert 3mal mit je 10 ml Methylenchlorid, wäscht die vereinigten Methylenchloridlösungen mit 10 ml gesättigter Natriumchloridlösung, trocknet die organische Phase über Natriumsulfat und engt die Lösung im Vakuum ein.

Der ölige Rückstand wird durch Säulenchromatographie gereinigt. Mit Ether/Aceton (2:1) erhält man 301 mg (92% d. Th.) der Titelverbindung als farbloses Öl, das bei Raumtemperatur langsam kristallisiert.

Schmelzpunkt: 96 bis 97,5°C

$C_{17}H_{26}O_3$: 278,395

$^{1}H$-NMR ($CDCl_3$): 5,48 (m, 2H), 2,88 (m, 2H).

$^{13}C$-NMR (Methanol-d4): 222,52, 134,76, 133,78, 79,01, 78,34, 58,49, 46,63, 45,04, 44,31, 43,52, 42,43, 36,28, 30,01, 27,70, 27,21.

d) *3-(m-Methoxycarbonylbenzyliden)-6β-(3'S-hydroxy-3'-cyclohexyl-1'E-propenyl)-7α-hydroxy-cis-bicyclo-[3.3.0]-octan*

Allgemeine Formel I: $R_1$ = $CH_3$; $R_2$ = Cyclohexyl

Zu einer Suspension von 2,141 g Natriumbis-(trimethylsilyl)amid in 20 ml absolutem Benzol gibt man unter Rühren bei Raumtemperatur sowie unter Ausschluss von Feuchtigkeit und unter Stickstoff als Schutzgas 5,743 g m-Methoxycarbonylbenzyltriphenylphosphoniumbromid. Man rührt 30 min bei Raumtemperatur nach und erhitzt 1 h zum leichten Sieden unter Rückfluss. Man lässt wieder auf Raumtemperatur abkühlen und gibt nun die Lösung von 224,3 mg des in Beispiel 1c erhaltenen Produktes in 5 ml absolutem Benzol

sowie 154 µl Thiophenol hinzu. Man rührt zunächst 1 h bei Raumtemperatur und dann eine Woche lang bei 50 bis 60°C (DC-Laufmittel: Ether/Essigsäureethylester 4:1).

Man verrührt das Reaktionsgemisch mit 100 ml Wasser, trennt die Benzolschicht ab, extrahiert die wässerige Schicht 3mal mit je 25 ml Ether, wäscht die vereinigten organischen Phasen mit 25 ml gesättigter Natriumchloridlösung, trocknet über Natriumsulfat und engt die Lösung im Vakuum ein.

Der ölige Rückstand wird durch Säulenchromatographie gereinigt. Mit Ether/Essigsäureethylester (4:1) erhält man 145 mg (= 44% d. Th.) der 3EZ-Form der Titelverbindung als farbloses Öl.

Eine Trennung in die Z- und E-Isomeren erreicht man durch HPLC an einer *reversed phase*-Säule (LiChrosorb RP 80 10 µm [Knaur] als Säulenmaterial, Säulendurchmesser 4,6 mm, Durchflussgeschwindigkeit 2 ml/min). Mit Methanol/Wasser (8:2) als Laufmittel erhält man aus 65 mg EZ-Gemisch 15,6 mg des Z-Isomeren und 28,2 mg des gewünschten E-Isomeren der Titelverbindung, das bei 126,5 bis 128°C schmilzt und folgende Spektrenwerte zeigt.

$C_{26}H_{34}O_4$: 410,559

$^1$H-NMR ($CDCl_3$): 7,77 (m, 2H); 7,32 (m, 2H); 6,37 (s verbreitert, 1H); 5,49 (m, 2H); 3,94 (s, 3H); 3,70 (m, 2H).

$^{13}$C-NMR (Methanol-d4): 148,39, 140,30, 134,82, 134,33, 134,09, 131,23, 130,31, 129,40, 127,94, 122,58, 78,58, 78,34, 57,76, 52,59, 45,71, 45,04, 42,79, 41,15, 39,87, 38,90, 30,13, 27,70, 27,21.

e) *3EZ-(m-Carboxybenzyliden)-6β-(3'S-hydroxy-3'-cyclohexyl-1'E-propenyl)-7α-hydroxy-cis-bicyclo-[3.3.0]-octan*

Allgemeine Formel I: $R_1$ = H; $R_2$ = Cyclohexyl

Zu einer Lösung von 145 mg des in Beispiel 1d erhaltenen Produktes in der 3EZ-Form in 5 ml Methanol gibt man 2 ml 1 N Natronlauge und rührt 2 h bei Raumtemperatur (DC-Laufmittel: Ether/Aceton 9:1). Man lässt über Nacht bei Raumtemperatur stehen, gibt unter Rühren 2,2 ml 1 N Salzsäure hinzu, extrahiert 3mal mit je 10 ml Methylenchlorid, wäscht die vereinigten Methylenchloridlösungen mit 10 ml gesättigter Natriumchloridlösung, trocknet über Natriumsulfat und engt die Lösung im Vakuum ein.

Man erhält 106 mg (= 76% d. Th.) der Titelverbindung als farbloses Öl, das bei Raumtemperatur langsam fest wird.

$C_{25}H_{32}O_4$: 396,532

$^1$H-NMR ($CDCl_3$): 7,85 (m, 2H), 7,37 (m, 2H), 6,33 (s verbreitert, 1H), 5,46 (m, 2H), 3,67 (m, 2H), 3,08 (s, 3H, mit $D_2O$ austauschbar).

f) Diese Verbindung kann z.B. wie folgt in ihr Natriumsalz ($R_1$ = Na) übergeführt werden:

Zu einer Lösung von 70,2 mg der Carboxyverbindung in 5 ml Methanol gibt man unter Rühren die Lösung von 29,8 mg Natriumhydrogencarbonat in 3 ml Wasser, lässt über Nacht stehen und erwärmt 1 h auf 60°C. Man engt die Lösung im Vakuum ein, gibt zum Rückstand weitere 10 ml Methanol und engt nochmals im Vakuum ein. Den trockenen Rückstand extrahiert man 3mal mit jeweils 5 ml Methanol, filtriert die methanolische Lösung und engt das Filtrat im Vakuum ein.

Man erhält 70,5 mg (= 95% d. Th.) des Natriumsalzes in Form leicht gelblicher Kristalle.

$C_{25}H_{31}O_4Na$: 418,514

$^1$H-NMR (Methanol-d4): 6,44 (s verbreitert, 1H), 5,49 (m, 2H).

*Beispiel 2:*

*3E-(m-Carboxybenzyliden)-6β-(3'S-hydroxy-3'-cyclohexyl-1'E-propenyl)-7α-hydroxy-cis-bicyclo-[3.3.0]-octan*

Allgemeine Formel I: $R_1$ = H; $R_2$ = Cyclohexyl

Aus 86 mg des nach Beispiel 1d erhaltenen Produktes in der 3E-Form in 12 ml Methanol und 3 ml 1 N Natronlauge erhält man in Analogie zu Beispiel 1e 78,5 mg (95% d. Th.) der Titelverbindung als farbloses Öl, das bei Raumtemperatur kristallisiert.

Schmelzpunkt: 161 bis 164°C

$C_{25}H_{32}O_4$: 396,532

$^1$H-NMR ($CDCl_3$): 7,90 (m, 2H), 7,43 (m, 2H), 6,37 (s verbreitert, 1H), 5,51 (m, 2H), 3,78 (m, 3H).

*Beispiel 3:*

a) *3,3-Ethylendioxy-6β-(3'-oxo-3'-[1''-adamantyl]-1'E-propenyl)-7α-tert.-butyldimethylsilyloxy-cis-bicyclo-[3.3:0]-octan*

Allgemeine Formel VI: $R_2$ = 1-Adamantyl; Z = tert.-Butyldimethylsilyl

Man setzt 242,6 mg Natriumhydriddispersion (50%ig in Öl) in 20 ml absolutem Toluol mit 1,47 g 2-Oxo-2-(1'-adamantyl)ethanphosphonsäuredimethylester in 40 ml absolutem Toluol sowie 1,5 g 3,3-Ethylendioxy-6β-formyl-7α-tert.-butyldimethylsilyloxy-cis-bicyclo-[3.3.0]-octan in 5 ml absolutem Toluol in Analogie zu Beispiel 1a miteinander um. Man rührt hier jedoch 6 h und lässt über Nacht im Kühlschrank stehen (DC-Laufmittel: Ether/Hexan 1:1).

Nach Säulenchromatographie mit Petrolether/Ether (4:1) erhält man 999,1 mg (= 45% d. Th.) der Titelverbindung.

$C_{29}H_{46}SiO_4$: 486,778

$^1$H-NMR ($CDCl_3$): 6,61 (m, 2H), 3,88 (s, 4H), 3,76 (m, 1H), 0,88 (s, 9H), 0,03 (s, 6H).

Der oben eingesetzte 2-Oxo-2-(1'-adamantyl)ethanphosphonsäuredimethylester wurde wie folgt erhalten:

Zu 45 ml trockenem Ether gibt man unter Rühren in einer Stickstoffatmosphäre bei etwa −70°C tropfenweise 77,5 ml einer 15%igen Lösung von n-Butyllithium in Hexan und gibt danach die Lösung von 10,8 ml Methanphosphansäuredimethylester in 50 ml trockenem Tetrahydrofuran zu. Man rührt 15 min nach, fügt tropfenweise die Lösung von 10,4 g 1-Adamantancarbonsäureethylester in 50 ml trockenem Tetrahydrofuran bei −75°C hinzu und rührt 3 h nach. Man lässt über Nacht auf 0°C kommen und stellt durch vorsichtige Zugabe von 4N Salzsäure bei 0°C auf pH 4 bis 5 ein.

Man engt die Lösung im Vakuum ein, nimmt den Rückstand in 100 ml Essigsäureethylester auf und

wäscht 3mal mit je 30 ml gesättigter Natriumchloridlösung. Die vereinigten Waschwässer extrahiert man 2mal mit je 10 ml Essigsäureethylester nach und gibt die organischen Phasen zusammen. Man trocknet über Natriumsulfat und engt die Lösung im Vakuum ein. Der ölige Rückstand wird im Vakuum destilliert, wobei man 10,4 g (= 72% d. Th.) des Produktes als farbloses Öl erhält.

Siedepunkt: 170 bis 171°C/0,2 mmHg

$^1$H-NMR ($CDCl_3$): 3,86 (s, 3H), 3,67 (s, 3H), 3,31 (s, 1H), 2,96 (s, 1H), 1,94 (m, 15H).

b) *3,3-Ethylendioxy-6β-(3'S-hydroxy-3'-[1"-adamantyl]-1'E-propenyl)-7α-tert.-butyldimethylsilyloxy-cis-bicyclo-[3.3.0]-octan*

Allgemeine Formel VIII: $R_2$ = 1-Adamantyl; Z = tert.-Butyldimethylsilyl

In Analogie zu Beispiel 1b erhält man aus 422,5 mg des Produktes von Beispiel 3a in 5 ml Methanol und 2,6 ml 0,4M methanolischer Cer-III-chloridlösung sowie 51,3 mg Natriumborhydrid nach Säulenchromatographie mit Petrolether/Ether (2:1) 314,1 mg (= 74% d. Th.) der Titelverbindung und 56,9 mg (= 13% d. Th.) des entsprechenden 3'R-Isomeren, das verworfen wird.

$C_{29}H_{48}SiO_4$: 488,794

3'S-Isomer:

$^1$H-NMR ($CDCl_3$): 5,50 (m, 2H), 3,93 (s, 4H), 3,70 (m, 2H), 0,93 (s, 9H), 0,08 (s, 6H).

$^{13}$C-NMR (Methanol-d4): 134,88, 130,37, 119,97, 82,05, 80,28, 65,37, 64,76, 58,07, 43,95, 43,10, 42,49, 40,91, 39,26, 38,29, 37,80, 36,77, 29,58, 26,48, 18,81.

c) *6β-(3'S-Hydroxy-3'-[1"-adamantyl]-1'E-propenyl)-7α-hydroxy-cis-bicyclo-[3.3.0]-octan-3-on*

Allgemeine Formel II: $R_2$ = 1-Adamantyl

Analog Beispiel 1c erhält man aus 314,1 mg des Produktes von Beispiel 3b nach Säulenchromatographie mit Ether/Aceton (3:1) 192,2 mg der Titelverbindung als weisse Kristalle.

Schmelzpunkt: 142 bis 143,5°C (aus Diisopropylether)

$C_{21}H_{30}O_3$: 330,471

$^1$H-NMR ($CDCl_3$): 5,52 (m, 2H), 3,95 (m, 1H), 3,53 (m, 1H).

d) *3EZ-(m-Methoxycarbonylbenzyliden)-6β-(3'S-hydroxy-3'-[1"-adamantyl]-1'E-propenyl)-7α-hydroxy-cis-bicyclo-[3.3.0]-octan*

Allgemeine Formel I: $R_1$ = $CH_3$; $R_2$ = 1-Adamantyl

In Analogie zu Beispiel 1d werden 195,9 mg des in Beispiel 3c gewonnenen Produktes, 2,91 g m-Methoxycarbonylbenzyltriphenylphosphoniumbromid und 1,09 g Natriumbis(trimethylsilyl)-amid miteinander umgesetzt. Nach Säulenchromatographie mit Ether/Aceton (4:1) erhält man 114,4 mg der Titelverbindung (= 42% d. Th.) als feste Substanz.

$C_{30}H_{38}O_4$: 462,636

$^1$H-NMR ($CDCl_3$): 7,86 (m, 2H), 7,36 (m, 2H), 6,35 (s verbreitert, 1H), 5,50 (m, 2H), 3,88 (s, 3H), 3,80 (m, 2H).

e) *3EZ-(m-Carboxybenzyliden)-6β-(3'S-hydroxy-3'-[1"-adamantyl]-1'E-propenyl)-7α-hydroxy-cis-bicyclo-[3.3.0]-octan*

Allgemeine Formel I: $R_1$ = H; $R_2$ = 1-Adamantyl

In Analogie zu Beispiel 1e erhält man aus 110,4 mg des nach Beispiel 3d gewonnenen Produktes in 10 ml Methanol mit 3 ml 1 N Natronlauge 79,3 mg der Titelverbindung (= 74% d. Th.) als feste Substanz.

$C_{29}H_{36}O_4$: 448,609

$^1$H-NMR ($CDCl_3$): 7,83 (m, 2H), 7,37 (m, 2H), 6,37 (s verbreitert, 1H), 5,48 (m, 2H), 3,47 (m, 2H), 3,25 (s, 3H, mit $D_2O$ austauschbar).

*Beispiel 4:*

Man verfährt wie in den Beispielen 1 bis 3 und erhält aus den entsprechenden Ausgangsmaterialien:

a) *3E-(m-Ethoxycarbonylbenzyliden)-6β-(3'S-hydroxy-3'-cyclohexyl-1'E-propenyl)-7α-hydroxy-cis-bicyclo-[3.3.0]-octan*

Allgemeine Formel I: $R_1$ = $C_2H_5$; $R_2$ = Cyclohexyl

$C_{27}H_{36}O_4$: 424,586

Schmelzpunkt: 115 bis 116°C

$^1$H-NMR ($CDCl_3$): 7,78 (m, 2H), 7,30 (m, 2H), 6,31 (s verbreitert, 1H), 5,48 (m, 2H), 4,23 (q, 2H), 3,70 (m, 2H), 1,38 (t).

b) *3E-(m-[n-Propyloxycarbonyl]benzyliden)-6β-(3'S-hydroxy-3'-cyclohexyl-1'E-propenyl)-7α-hydroxy-cis-bicyclo-[3.3.0]-octan*

Allgemeine Formel I: $R_1$ = n-$C_3H_7$; $R_2$ = Cyclohexyl

$C_{28}H_{38}O_4$: 438,613

Schmelzpunkt: 68 bis 71°C

$^1$H-NMR ($CDCl_3$): 7,78 (m, 2H), 7,32 (m, 2H), 6,33 (s verbreitert, 1H), 5,43 (m, 2H), 4,23 (t, 2H), 3,64 (m, 2H).

c) *Natriumsalz des 3E-(m-Carboxybenzyliden)-6β-(3'S-hydroxy-3'-cyclohexyl-1'E-propenyl)-7α-hydroxy-cis-bicyclo-[3.3.0]-octans*

Allgemeine Formel I: $R_1$ = Na; $R_2$ = Cyclohexyl

$C_{25}H_{31}O_4Na$: 418,514

$^1$H-NMR (Methanol-d4): 6,44 (s verbreitert, 1H), 5,51 (m, 2H).

d) *3E-(m-Carboxybenzyliden)-6β-(3'S-hydroxy-3'-[adamantyl-1"]-1'E-propenyl)-7α-hydroxy-cis-bicyclo-[3.3.0]-octan*

Allgemeine Formel I: $R_1$ = H; $R_2$ = Adamantyl

$C_{29}H_{36}O_4$: 448,609

$^1$H-NMR (Methanol-d4): 7,78 (m, 2H), 7,37 (m, 2H), 6,38 (s verbreitert, 1H), 5,50 (m, 2H).

e) *3EZ-(m-Carboxybenzyliden)-6β-(3'S-hydroxy-3'-[3",5"-dimethyladamantyl-1"]-1'E-propenyl)-7α-hydroxy-cis-bicyclo-[3.3.0]-octan*

Allgemeine Formel I: $R_1$ = H; $R_2$ = 3,5-Dimethyladamantyl-1

f) *3E-(m-[tert.-Butyloxycarbonyl]-benzyliden)-6β-(3'S-hydroxy-3'-cyclohexyl-1'E-propenyl)-7α-hydroxy-cis-bicyclo-[3.3.0]-octan*

Allgemeine Formel I: $R_1$ = $(CH_3)_3C-$; $R_2$ = Cyclohexyl

g) *3E-(m-Carboxybenzyliden)-6β-(3'S-hydroxy-3'-[4''-methylcyclohexyl]-1'E-propenyl)-7α-hydroxy-cis-bicyclo-[3.3.0]-octan*
Allgemeine Formel I: $R_1$ = H; $R_2$ = 4-Methylcyclohexyl

h) *Kaliumsalz des 3E-(m-Carboxybenzyliden)-6β-(3'S-hydroxy-3'-cyclohexyl-1'E-propenyl)-7α-hydroxy-cis-bicyclo-[3.3.0]-octans*
Allgemeine Formel I: $R_1$ = K; $R_2$ = Cyclohexyl
und weitere Verbindungen der Formel I.

**Patentansprüche** für die Vertragsstaaten BE, CH, DE, FR, GB, IT, LI, LU, NL, SE

1. Neue cis-Bicyclo-[3.3.0]-octanderivate der Formel (I)

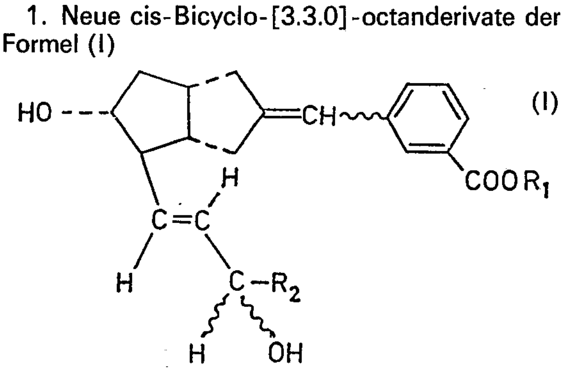
(I)

in denen der Phenylrest in bezug auf die Doppelbindung die EZ- oder E-Konfiguration aufweisen kann und, am den Rest $R_2$ tragenden Kohlenstoffatom, die RS- oder S-Konfiguration vorliegen kann, und worin

$R_1$ ein Wasserstoffatom, einen geradkettigen oder verzweigten Alkylrest mit 1 bis 4 Kohlenstoffatomen, oder ein pharmazeutisch verträgliches Kation bedeutet, und

$R_2$ einen Cyclohexylrest, einen 4-Methylcyclohexylrest oder einen 1-Adamantylrest der Struktur

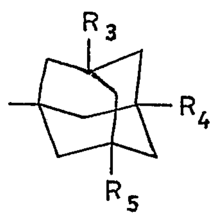

in der $R_3$, $R_4$ und $R_5$ gleich oder verschieden sind und für Wasserstoff oder Methyl stehen, bedeutet.

2. Neue cis-Bicyclo-[3.3.0]-octanderivate der Formel

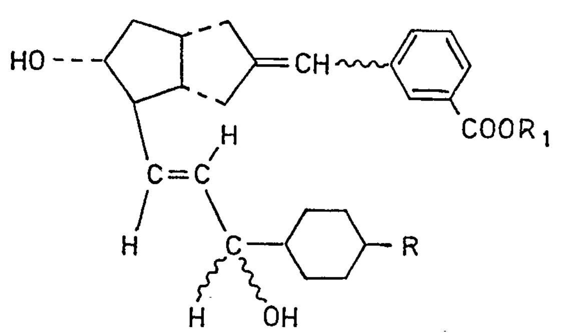

worin $R_1$ die gleiche Bedeutung wie in Formel (I) hat, R für Wasserstoff oder Methyl steht, und in denen der Phenylrest in bezug auf die Doppelbindung die EZ- oder E-Konfiguration aufweisen kann und, am mit dem Cyclohexylrest verbundenen Kohlenstoffatom 3', die RS- oder S-Konfiguration vorliegen kann.

3. Neue cis-Bicyclo-[3.3.0]-octanderivate gemäss den Ansprüchen 1 und 2, in denen $R_1$ für einen Methyl- oder einen Ethylrest steht.

4. Neue cis-Bicyclo-[3.3.0]-octanderivate gemäss den Ansprüchen 1 und 2, in denen $R_1$ für ein Natrium- oder ein Kaliumion steht.

5. Die 3'S-Formen der neuen cis-Bicyclo-[3.3.0]-octanderivate gemäss den Ansprüchen 1 bis 4.

6. 3-(m-Carboxybenzyliden)-6β-(3'S-hydroxy-3'-cyclohexyl-1'E-propenyl)-7α-hydroxy-cis-bicyclo-[3.3.0]-octan der Formel

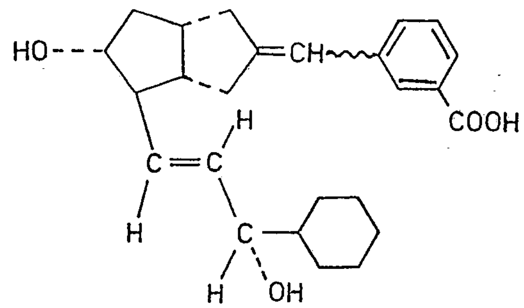

in der 3EZ- oder 3E-Konfiguration und dessen pharmazeutisch verträgliche Salze, insbesondere dessen Natriumsalz.

7. Arzneimittel, dadurch gekennzeichnet, dass es als Wirkstoff wenigstens ein cis-Bicyclo-[3.3.0]-octanderivat entsprechend den Ansprüchen 1 bis 6 enthält.

8. Arzneimittel gemäss Anspruch 7, dadurch gekennzeichnet, dass es als Wirkstoff pro Einzeldosis 0,01 bis 50 mg eines cis-Bicyclo-[3.3.0]-octanderivates entsprechend den Ansprüchen 1 bis 6 enthält.

9. Arzneimittel gemäss den Ansprüchen 7 und 8, dadurch gekennzeichnet, dass es zur parenteralen Applikation geeignet ist und 0,01 bis 10 mg eines cis-Bicyclo-[3.3.0]-octanderivates entsprechend den Ansprüchen 1 bis 6 enthält.

10. Arzneimittel gemäss Anspruch 9 zur parenteralen Applikation, dadurch gekennzeichnet, dass es in einem flüssigen, sterilen Träger 0,01 bis 10 mg eines cis-Bicyclo-[3.3.0]-octanderivates entsprechend den Ansprüchen 1 bis 6 in gelöster oder suspendierter Form enthält.

11. Arzneimittel gemäss den Ansprüchen 8 und 9, dadurch gekennzeichnet, dass es zur oralen Applikation geeignet ist und 0,1 bis 50 mg eines cis-Bicyclo-[3.3.0]-octanderivates entsprechend den Ansprüchen 1 bis 6 enthält.

12. Arzneimittel gemäss Anspruch 11 zur oralen Applikation, dadurch gekennzeichnet, dass es in Form von Tabletten, Dragees oder Kapseln vorliegt.

13. Arzneimittel gemäss Anspruch 7, dadurch gekennzeichnet, dass es in Form von Tabletten,

Dragees oder Kapseln mit verzögerter Wirkstofffreigabe vorliegt und pro Einzeldosis bis zu 250 mg eines cis-Bicyclo-[3.3.0]-octanderivates entsprechend den Ansprüchen 1 bis 6 enthält.

14. Verfahren zur Herstellung von neuen cis-Bicyclo-[3.3.0]-octanderivaten der Formel (I)

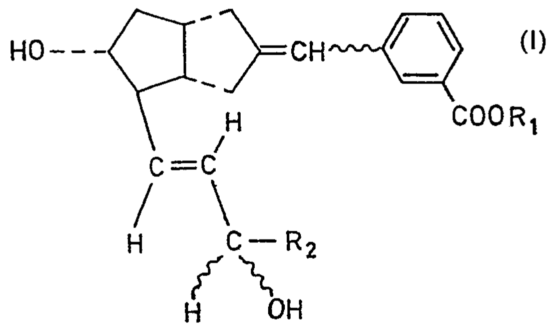

(I)

in denen der Phenylrest in bezug auf die Doppelbindung die EZ- oder E-Konfiguration aufweisen kann und, am den Rest $R_2$ tragenden Kohlenstoffatom, die RS- oder S-Konfiguration vorliegen kann, und worin

$R_1$ ein Wasserstoffatom, einen geradkettigen oder verzweigten Alkylrest mit 1 bis 4 Kohlenstoffatomen, oder ein pharmazeutisch verträgliches Kation bedeutet,

$R_2$ einen Cyclohexylrest, einen 4-Methylcyclohexylrest oder einen 1-Adamantylrest der Struktur

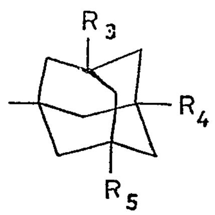

in der $R_3$, $R_4$ und $R_5$ gleich oder verschieden sind und für Wasserstoff oder Methyl stehen, bedeutet, dadurch gekennzeichnet, dass man eine Verbindung der allgemeinen Formel (II)

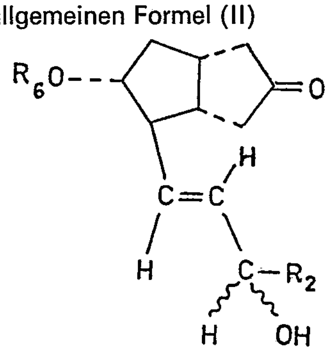

(II)

worin $R_2$ die gleiche Bedeutung wie oben hat und $R_6$ für ein Wasserstoffatom oder eine unter milden Bedingungen abspaltbare Schutzgruppe steht, unter Ausschluss von Feuchtigkeit und Sauerstoff in Gegenwart aprotischer Lösungsmittel bei Temperaturen von etwa 0 bis etwa 100°C mit einer Verbindung der Formel (III)

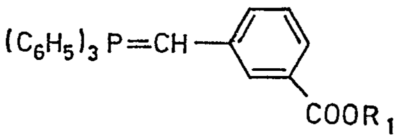

(III)

worin $R_1$ die gleiche Bedeutung wie oben hat, umsetzt und, wenn $R_6$ von Wasserstoff verschieden ist, anschliessend die Schutzgruppe $R_6$ abspaltet.

15. Verfahren gemäss Anspruch 14, dadurch gekennzeichnet, dass man die Reaktion unter Zusatz einer schwach sauren Verbindung, insbesondere einem gegebenenfalls substituierten Thiophenol, durchführt.

16. Verfahren gemäss den Ansprüchen 14 und 15, dadurch gekennzeichnet, dass man in der erhaltenen Verbindung der Formel (I), wenn darin $R_1$ einen Alkylrest darstellt, die Gruppe $COOR_1$ zur Carboxylgruppe verseift und das so erhaltene Produkt (mit $R_1$ = H) gegebenenfalls anschliessend in ein pharmazeutisch verträgliches Salz überführt.

17. Verfahren zur Herstellung der Arzneimittel gemäss den Ansprüchen 7 bis 13, dadurch gekennzeichnet, dass man die cis-Bicyclo-[3.3.0]-octanderivate entsprechend den Ansprüchen 1 bis 6 in üblicher Weise mit Trägermaterialien, Verdünnungsstoffen sowie gegebenenfalls Bindemitteln, Tablettensprengstoffen und anderen üblichen Hilfsstoffen verarbeitet und aus dem erhaltenen Gemisch die Einzeldosierungsformen fertigt.

**Patentansprüche** für den Vertragsstat: AT

1. Verfahren zur Herstellung von neuen cis-Bicyclo-[3.3.0]-octanderivaten der Formel (I)

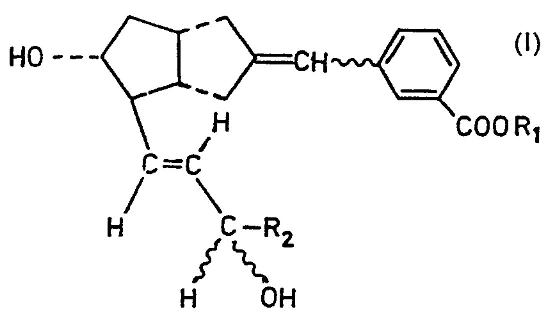

(I)

in denen der Phenylrest in bezug auf die Doppelbindung die EZ- oder E-Konfiguration aufweisen kann und, am den Rest $R_2$ tragenden Kohlenstoffatom, die RS- oder S-Konfiguration vorliegen kann, und worin

$R_1$ ein Wasserstoffatom, einen geradkettigen oder verzweigten Alkylrest mit 1 bis 4 Kohlenstoffatomen, oder ein pharmazeutisch verträgliches Kation bedeutet, und

$R_2$ einen Cyclohexylrest, einen 4-Methylcyclohexylrest oder einen 1-Adamantylrest der Struktur

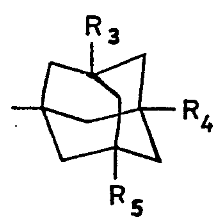

in der $R_3$, $R_4$ und $R_5$ gleich oder verschieden sind und für Wasserstoff oder Methyl stehen, bedeutet,

dadurch gekennzeichnet, dass man eine Verbindung der allgemeinen Formel (II)

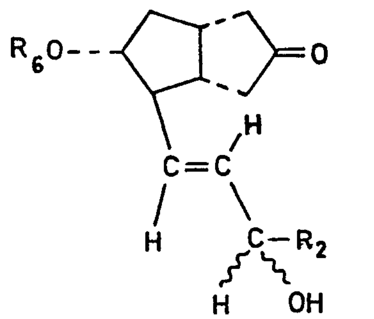

(II)

worin $R_2$ die gleiche Bedeutung wie oben hat und $R_6$ für ein Wasserstoffatom oder eine unter milden Bedingungen abspaltbare Schutzgruppe steht, unter Ausschluss von Feuchtigkeit und Sauerstoff in Gegenwart aprotischer Lösungsmittel bei Temperaturen von etwa 0 bis etwa 100°C mit einer Verbindung der Formel (III)

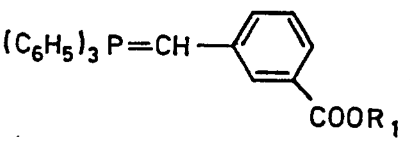

(III)

worin $R_1$ die gleiche Bedeutung wie oben hat, umsetzt und, wenn $R_6$ von Wasserstoff verschieden ist, anschliessend die Schutzgruppe $R_6$ abspaltet.

2. Verfahren gemäss Anspruch 1, dadurch gekennzeichnet, dass man die Reaktion unter Zusatz einer schwach sauren Verbindung, insbesondere einem gegebenenfalls substituierten Thiophenol, durchführt.

3. Verfahren gemäss den Ansprüchen 1 und 2, dadurch gekennzeichnet, dass $R_6$ für eine tert.-Butyldimethylsilylgruppe steht.

4. Verfahren gemäss den Ansprüchen 1 bis 3, dadurch gekennzeichnet, dass man in der erhaltenen Verbindung der Formel (I), wenn darin $R_1$ einen Alkylrest darstellt, die Gruppe $COOR_1$ zur Carboxylgruppe verseift und das so erhaltene Produkt (mit $R_1$ = H) gegebenenfalls anschliessend in ein pharmazeutisch verträgliches Salz überführt.

**Claims** for the Contracting States BE, CH, DE, FR, GB, IT, LI, LU, NL, SE

1. New cis-bicyclo-[3.3.0]-octane derivatives corresponding to the Formula (I)

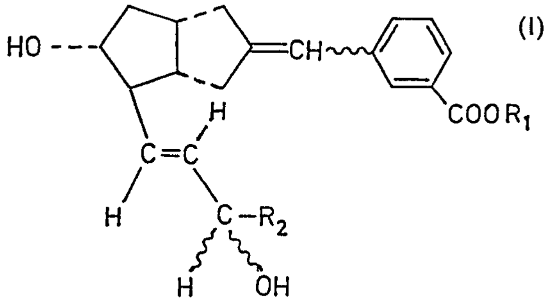

(I)

in which the phenyl radical may have the EZ- or Z-configuration in relation to the double bond whilst the RS- or S-configuration may be present on the carbon atom carrying the radical $R_2$, and in which

$R_1$ represents a hydrogen atom, a straight-chain or branched alkyl radical containing from 1 to 4 carbon atoms or a pharmaceutically acceptable cation, and

$R_2$ represents a cyclohexyl radical, a 4-methylcyclohexyl radical or a 1-adamantyl radical having the following structure

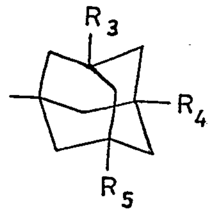

in which $R_3$, $R_4$ and $R_5$ may be the same or different and represent hydrogen or methyl.

2. New cis-bicyclo-[3.3.0]-octane derivatives corresponding to the following formula

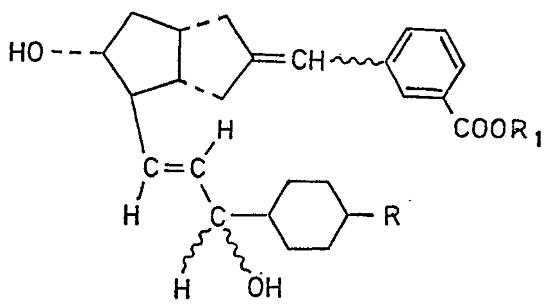

in which $R_1$ has the same meaning as in Formula (I), R represents hydrogen or methyl, and in which the phenyl radical may have the EZ- or E-configuration in relation to the double bond whilst the 3'RS- or S-configuration may be present on the carbon atom attached to the cyclohexyl radical.

3. New cis-bicyclo-[3.3.0]-octane derivatives as claimed in Claims 1 and 2 in which $R_1$ represents a methyl or an ethyl radical.

4. New cis-bicyclo-[3.3.0]-octane derivatives as claimed in Claims 1 and 2 in which $R_1$ represents a sodium or a potassium ion.

5. The 3'S-forms of the new cis-bicyclo-[3.3.0]-octane derivatives claimed in Claims 1 to 4.

6. 3-(m-Carboxybenzylidene)-6β-(3'S-hydroxy-3'-cyclohexyl-1'E-propenyl)-7α-hydroxy-cis-bicyclo-[3.3.0]-octane corresponding to the following formula

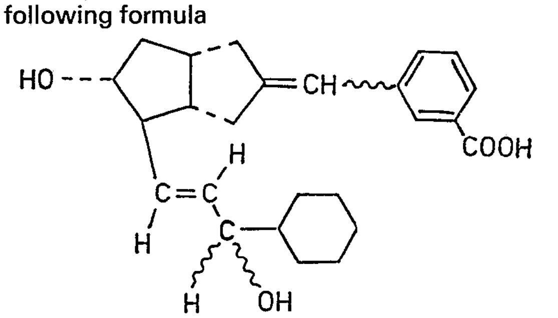

in the 3EZ- or 3E-configuration and its pharmaceutically acceptable salts, particularly its sodium salt.

7. A medicament, characterized in that it contains as active principle at least one cis-bicyclo-[3.3.0]-octane derivative of the type claimed in Claims 1 to 6.

8. A medicament as claimed in Claim 7, characterized in that it contains as active principle from 0.01 to 50 mg per single dose of a cis-bicyclo-[3.3.0]-octane derivative of the type claimed in Claims 1 to 6.

9. A medicament as claimed in Claims 7 and 8, characterized in that it is suitable for parenteral application and contains from 0.01 to 10 mg of a cis-bicyclo-[3.3.0]-octane derivative of the type claimed in Claims 1 to 6.

10. A medicament as claimed in Claim 9 for parenteral application, characterized in that it contains from 0.01 to 10 mg of a cis-bicyclo-[3.3.0]-octane derivative of the type claimed in Claims 1 to 6 in dissolved or suspended form in a liquid, sterile vehicle.

11. A medicament as claimed in Claims 8 and 9, characterized in that it is suitable for oral application and contains from 0.1 to 50 mg of a cis-bicyclo-[3.3.0]-octane derivative of the type claimed in Claims 1 to 6.

12 A medicament as claimed in Claim 11 for oral application, characterized in that it is in the form of tablets, dragees or capsules.

13. A medicament as claimed in Claim 7, characterized in that it is in the form of sustained-release tablets, dragees or capsules and contains up to 250 mg per individual dose of a cis-bicyclo-[3.3.0]-octane derivative of the type claimed in Claims 1 to 6.

14. A process for the production of new cis-bicyclo-[3.3.0]-octane derivatives corresponding to the Formula (I)

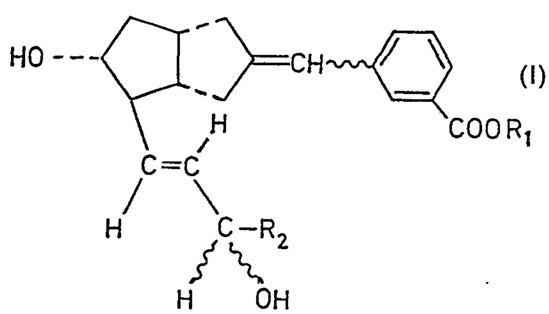

(I)

in which the phenyl radical may have the EZ- or E-configuration in relation to the double bond whilst the RS- or S-configuration may be present on the carbon atom carrying the radical $R_2$, and in which

$R_1$ represents a hydrogen atom, a straight-chain or branched alkyl radical containing from 1 to 4 carbon atoms or a pharmaceutically acceptable cation, and

$R_2$ represents a cyclohexyl radical, a 4-methylcyclohexyl radical or a 1-adamantyl radical having the following structure

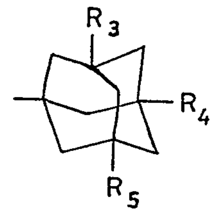

in which $R_3$, $R_4$ and $R_5$ may be the same or different and represent hydrogen or methyl, characterized in that a compound corresponding to the general Formula (II)

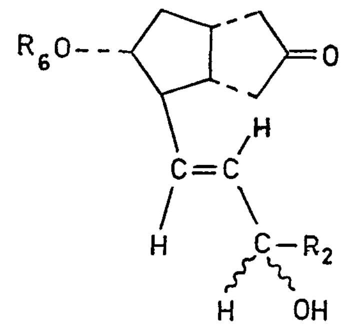

(II)

in which $R_2$ is as defined above and $R_6$ represents a hydrogen atom or a protective group which can be eliminated under mild conditions,
is reacted at about 0 to about 100°C with a compound corresponding to the Formula (III)

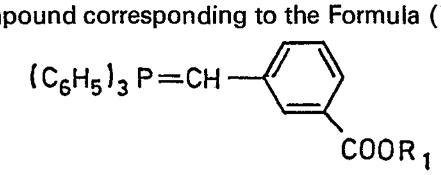

(III)

in which $R_1$ is as defined above,
in the presence of aprotic solvents and in the absence of moisture and oxygen and, where $R_6$ is different from hydrogen, the protective group $R_6$ is subsequently eliminated.

15. A process as claimed in Claim 14, characterized in that the reaction is carried out in the presence of a weakly acidic compound, more particularly an optionally substituted thiophenol.

16. A process as claimed in Claims 14 and 15, characterized in that in the compound obtained corresponding to Formula (I), when $R_1$ therein is an alkyl radical, the group $COOR_1$ is hydrolyzed to form the carboxyl group and the product thus obtained (with $R_1$ = H) is then optionally converted into a pharmaceutically acceptable salt.

17. A process for producing the medicaments claimed in Claims 7 to 13, characterized in that the cis-bicyclo-[3.3.0]-octane derivatives claimed in Claims 1 to 6 are processed in the usual way with carrier materials, diluents and, optionally, binders, tablet disintegrating agents and other standard auxiliaries and the individual dosage forms are prepared from the mixture obtained.

**Claims** for the Contracting State: AT

1. A process for the production of new cis-bicyclo-[3.3.0]-octane derivatives corresponding to the Formula (I)

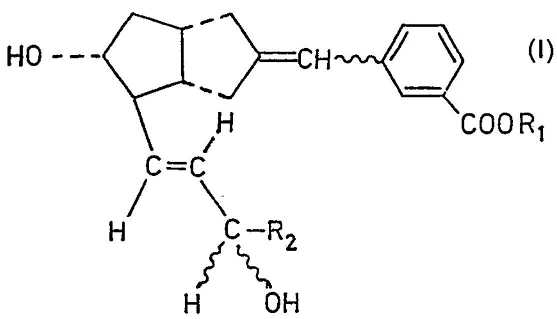

(I)

in which the phenyl radical may have the EZ- or E-configuration in relation to the double bond whilst the RS- or S-configuration may be present on the carbon atom carrying the radical $R_2$, and in which

$R_1$ represents a hydrogen atom, a straight-chain or branched alkyl radical containing from 1 to 4 carbon atoms or a pharmaceutically acceptable cation, and

$R_2$ represents a cyclohexyl radical, a 4-methylcyclohexyl radical or a 1-adamantyl radical having the following structure

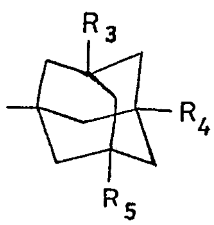

in which $R_3$, $R_4$ and $R_5$ are the same or different and represent hydrogen or methyl,
characterized in that a compound corresponding to the general Formula (II)

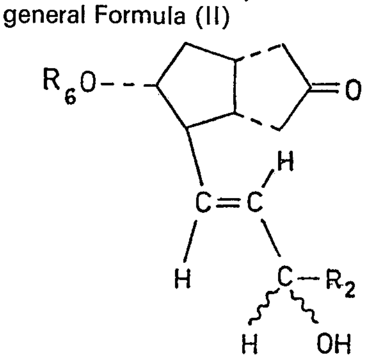

(II)

in which $R_2$ is as defined above and $R_6$ represents a hydrogen atom or a protective group which can be eliminated under mild conditions,
is reacted at about 0 to about 100°C with a compound corresponding to the general Formula (III)

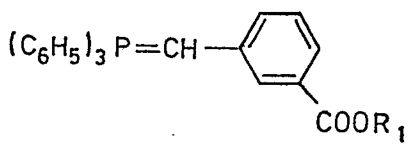

(III)

in which $R_1$ is as defined above,
in the presence of aprotic solvents and in the absence of moisture and oxygen and, where $R_6$ is different from hydrogen, the protective group $R_6$ is subsequently eliminated.

2. A process as claimed in Claim 1, characterized in that the reaction is carried out in the presence of a weakly acidic compound, more especially an optionally substituted thiophenol.

3. A process as claimed in Claims 1 and 2, characterized in that $R_6$ represents a tert.-butyldimethylsilyl group.

4. A process as claimed in Claims 1 to 3, characterized in that in the compound obtained corresponding to Formula (I), when $R_1$ therein is an alkyl radical, the group $COOR_1$ is hydrolyzed to form the carboxyl group and the compound obtained (with $R_1$ = H) is then optionally converted into a pharmaceutically acceptable salt.

**Revendications** pour les Etats contractants BE, CH, DE, FR, GB, IT, LI, LU, NL, SE

1. Nouveaux dérivés de cisbicyclo-[3.3.0]-octane de formule (I)

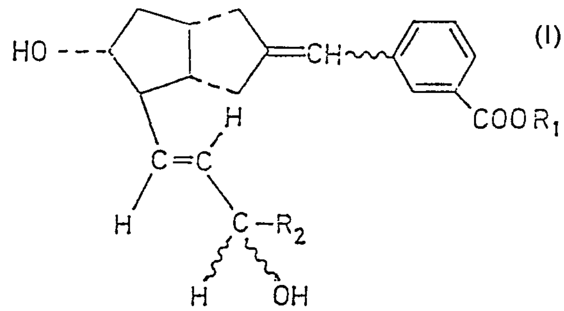

(I)

dans lesquels le reste phényle peut présenter, par rapport à la double liaison, la configuration EZ ou E et il peut y avoir, au niveau de l'atome de carbone portant le reste $R_2$, la configuration RS ou S, et

$R_1$ est un atome d'hydrogène, un reste alkyle à chaîne droite ou ramifiée ayant 1 à 4 atomes de carbone, ou un cation compatible du point de vue pharmaceutique, et

$R_2$ représente un reste cyclohexyle, un reste 4-méthylcyclohexyle ou un reste 1-adamantyle de formule

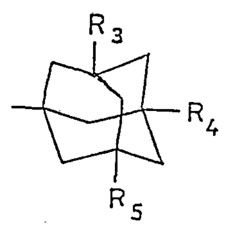

dans laquelle $R_3$, $R_4$ et $R_5$ sont égaux ou différents et représentent l'hydrogène ou un groupe méthyle.

2. Nouveaux dérivés de cisbicyclo-[3.3.0]-octane de formule

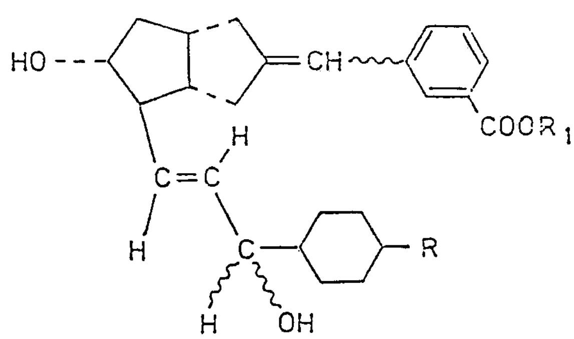

dans laquelle $R_1$ a la même définition que dans la formule (I), R est l'hydrogène ou le groupe méthyle, et dans lesquels le reste phényle peut présenter la configuration EZ ou E, par rapport à la double liaison, et la configuration RS ou S peut exister au niveau de l'atome de carbone 3' lié au reste cyclohexyle.

3. Nouveaux dérivés de cisbicyclo-[3.3.0]-octane suivant les revendications 1 et 2, dans lesquels $R_1$ représente un reste méthyle ou un reste éthyle.

4. Nouveaux dérivés de cisbicyclo-[3.3.0]-octane suivant les revendications 1 et 2, dans lesquels $R_1$ est un ion sodium ou un ion potassium.

5. Les formes 3'S des nouveaux dérivés de cisbicyclo-[3.3.0]-octane suivant les revendications 1 à 4.

6. Le 3-(m-carboxybenzylidène)-6β-(3'S-hydroxy-3'-cyclohexyl-1'E-propényl)-7α-hydroxy-cisbicyclo-[3.3.0]-octane de formule

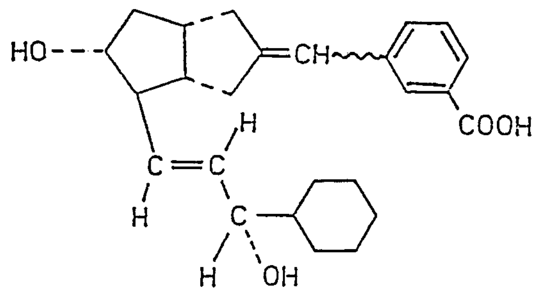

avec la configuration 3EZ ou 3E, et ses sels compatibles du point de vue pharmaceutique, notamment son sel de sodium.

7. Médicament, caractérisé en ce qu'il contient comme substance active au moins un dérivé cisbicyclo-[3.3.0]-octane suivant les revendications 1 à 6.

8. Médicament suivant la revendication 7, caractérisé en ce qu'il contient comme substance active, par dose individuelle, 0,01 à 50 mg d'un dérivé de cisbicyclo-[3.3.0]-octane suivant les revendications 1 à 6.

9. Médicament suivant les revendications 7 et 8, caractérisé en ce qu'il convient à l'administration parentérale et contient 0,01 à 10 mg d'un dérivé de cisbicyclo-[3.3.0]-octane suivant les revendications 1 à 6.

10. Médicament suivant la revendication 9, destiné à l'administration parentérale, caractérisé en ce qu'il contient, dans un véhicule liquide stérile, 0,01 à 10 mg d'un dérivé de cisbicyclo-[3.3.0]-octane suivant les revendications 1 à 6 sous forme de solution ou de suspension.

11. Médicament suivant les revendications 8 et 9, caractérisé en ce qu'il convient à l'administration orale et en ce qu'il contient 0,1 à 50 mg d'un dérivé de cisbicyclo-[3.3.0]-octane suivant les revendications 1 à 6.

12. Médicament suivant la revendication 11, destiné à l'administration orale, caractérisé en ce qu'il se présente sous forme de comprimés, de dragées ou de capsules.

13. Médicament suivant la revendication 7, caractérisé en ce qu'il se présente sous forme de comprimés, de dragées ou de capsules à libération retardée de la substance active et en ce qu'il contient, par dose individuelle, jusqu'à 250 mg d'un dérivé de cisbicyclo-[3.3.0]-octane suivant les revendications 1 à 6.

14. Procédé de production de dérivés nouveaux de cisbicyclo-[3.3.0]-octane de formule (I)

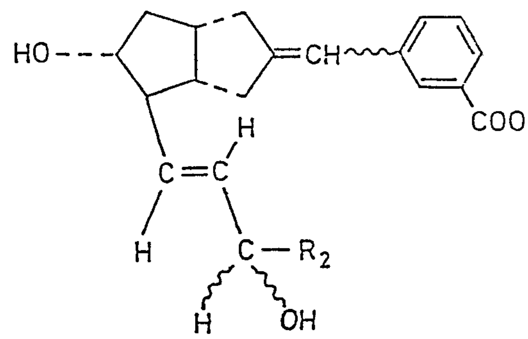

(I)

dans lesquels le reste phényle peut présenter, par rapport à la double liaison, la configuration EZ ou E et il peut y avoir, au niveau de l'atome de carbone portant le reste $R_2$, la configuration RS ou S, et

$R_1$ est un atome d'hydrogène, un reste alkyle à chaîne droite ou ramifiée ayant 1 à 4 atomes de carbone, ou un cation compatible du point de vue pharmaceutique, et

$R_2$ représente un reste cyclohexyle, un reste 4-méthylcyclohexyle ou un reste 1-adamantyle de formule

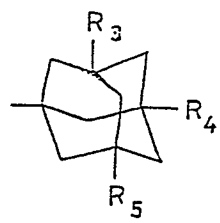

dans laquelle $R_3$, $R_4$ et $R_5$ sont égaux ou différents et représentent l'hydrogène ou un groupe méthyle, caractérisé en ce qu'on fait réagir un composé de formule générale (II)

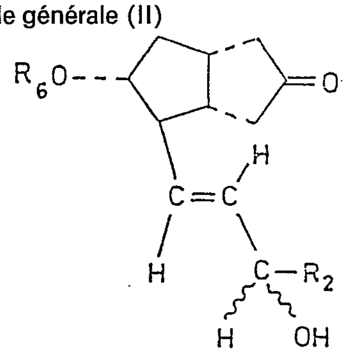

(II)

dans laquelle $R_2$ a la même définition que ci-dessus et $R_6$ est un atome d'hydrogène ou un groupe protecteur pouvant être éliminé dans des conditions douces,

à l'abri de l'humidité et de l'oxygène en présence de solvants aprotiques à des températures allant d'environ 0 à environ 100° C, avec un composé de formule (III)

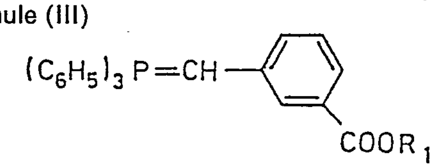

(III)

dans laquelle $R_1$ a la même définition que ci-dessus et, lorsque $R_6$ est différent de l'hydrogène, on élimine ensuite le groupe protecteur $R_6$.

15. Procédé suivant la revendication 14, caractérisé en ce qu'on conduit la réaction avec addition d'un composé faiblement acide, notamment un thiophénol éventuellement substitué.

16. Procédé suivant les revendications 14 et 15, caractérisé en ce qu'on saponifie le groupe $COOR_1$ en groupe carboxyle dans le composé obtenu de formule (I) lorsque $R_1$ y représente un reste alkyle, puis on transforme éventuellement en un sel acceptable du point de vue pharmaceutique le produit ainsi obtenu (avec $R_1$ = H).

17. Procédé de production des médicaments suivant les revendications 7 à 13, caractérisé en ce qu'on met en œuvre les dérivés de cisbicyclo-[3.3.0]-octane suivant les revendications 1 à 6 d'une manière classique avec des supports, des diluants ainsi que, le cas échéant, des liants, des agents de désintégration de comprimés et d'autres adjuvants classiques et on prépare, à partir du mélange obtenu, les formes posologiques unitaires.

**Revendications** pour l'Etat contractant: AT

1. Procédé de production de nouveaux dérivés de cisbicyclo-[3.3.0]-octane de formule (I)

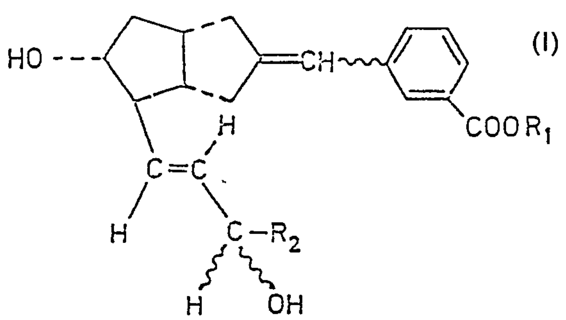

(I)

dans lesquels le reste phényle peut présenter, par rapport à la double liaison, la configuration EZ ou E et il peut y avoir, au niveau de l'atome de carbone portant le reste $R_2$, la configuration RS ou S, et

$R_1$ est un atome d'hydrogène, un reste alkyle à chaîne droite ou ramifiée ayant 1 à 4 atomes de carbone, ou un cation compatible du point de vue pharmaceutique, et

$R_2$ représente un reste cyclohexyle, un reste 4-méthylcyclohexyle ou un reste 1-adamantyle de formule

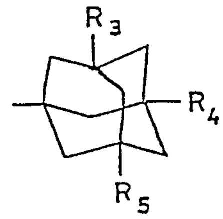

dans laquelle $R_3$, $R_4$ et $R_5$ sont égaux ou différents et représentent l'hydrogène ou un groupe méthyle, caractérisé en ce qu'on fait réagir un composé de formule générale (II)

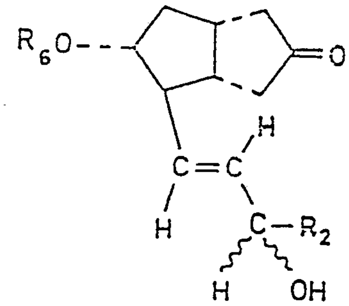

(II)

dans laquelle $R_2$ a la même définition que ci-dessus et $R_6$ est un atome d'hydrogène ou un groupe protecteur pouvant être éliminé dans des conditions douces,

à l'abri de l'humidité et de l'oxygène en présence de solvants aprotiques à des températures allant d'environ 0 à environ 100°C, avec un composé de formule (III)

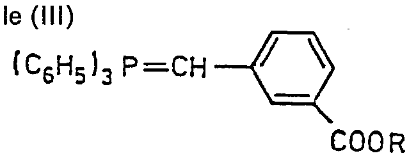

(III)

dans laquelle $R_1$ a la même définition que ci-dessus et, lorsque $R_6$ est différent de l'hydrogène, on élimine ensuite le groupe protecteur $R_6$.

2. Procédé suivant la revendication 1, caractérisé en ce qu'on conduit la réaction avec l'addition d'un composé faiblement acide, notamment un thiophénol éventuellement substitué.

3. Procédé suivant les revendications 1 et 2, caractérisé en ce que $R_6$ représente un groupe tertiobutyldiméthylsilyle.

4. Procédé suivant les revendications 1 à 3, caractérisé en ce qu'on saponifie le groupe $COOR_1$ en groupe carboxyle dans le composé obtenu de formule (I) lorsque $R_1$ y représente un reste alkyle, puis on transforme éventuellement en un sel pharmaceutiquement acceptable le produit ainsi obtenu (avec $R_1$ = H).